# EUROPEAN PATENT APPLICATION

(11) **EP 0 567 391 A1**
(43) Date of publication of application: **27.10.1993**
(21) Application number: 93401021.6
(22) Date of filing: 20.04.1993
(51) Int. Cl.: A61L 27/00, A61K 37/02

(54) **A biodegradable tgf-beta delivery system for bone regeneration**

(30) Priority: 24.04.1992 US 873266
(71) Applicant: Bristol-Myers Squibb Company, New York, N.Y. 10022 (US)
(72) Inventor: Wayne, Gombotz, Kirkland, WA 98034 (US)
(74) Representative: Beauchamps, Georges

(57) **Abstract**

A biodedegradable composition for the con- troled release of TGF-β is described. This composition is very useful for the delivery of TGF-β in the regeneration of bone. The composition contains, among other components, demineralized bone matrix and a degradable homopolymer or copolymer such as poly (lactic-co-glycolic acid). An implantable delivery system for TGF-β release in a predictable and controlled manner with time is described in which an implantable support is formed or coated with this biodegradable composition. TGF-β is stabilized in a composition contains an organic acid buffer, and to which additional components such as mannitol, human serum albumin, gelatin and/or a non-ionic detergent have been added.

## Description

### TECHNICAL FIELD OF THE INVENTION

The present invention is directed to a biodegradable composition for the release of TGF-β and delivery system composed of an implantable support coated with this composition, which releases TGF-β in a predictable and controlled manner with time. A composition for stabilizing TGF-β is described.

### BACKGROUND OF THE INVENTION

Bone has a remarkable capacity for regenerative growth; however, there are many clinical indications in which the bony repair process is impaired. These include situation in which there is too much bone loss for the bone to regenerate such as skeletal deformations caused by trauma, malformation or cancer or in reconstructive cosmetic surgerie. There is a critical need for the development of implant technology for use in numerous clinical situations to augment or promote bone healing.

Autogenous cartilage and bone are the materials of choice for skeletal augmentation or reconstruction. However, there are several drawbacks to using these materials including donor site morbidity, limited quantities of grafting materials and unpredictable implant resorption. As a result, a variety of different materials have been studied that could function as artificial for bone repair. These include ceramics, composites, bone derivatives and natural and synthetic polymers (C.J. Damien and J.R. Parsons (1991) J. Appl. Bio- mat., 2:187-208).

The ideal implant or graft material must possess certain basic properties. It should be biocompatible, sterilizable, exhibit osteoinduction (the stimulation of phenotypic conversion of mesenchymal cells into osteoblasts, with bone formation) and osteoconduction (acting as a trellis for new bone formation). Porosity and pore density of the implant play an important role in its osteoconductive properties. The material should also degrade in concert with new bone growth without interfering with the formation of new bone. The implant should neither induce adverse local tissue reaction nor be immunogenic or systemically toxic. Many of the materials presently under investigation only partially satisfy these requirements.

Poly (lactic acid) (PLA) and poly (lactic-co-glycolic acid) (PLPG) polymers have been used experimentally for osseous repair. They are both biocompatible and biodegradable and in some cases have been shown to induce bony wound healing (Hollinger, J.O. (1983) J. Biomed. Mater. Res., 17:71:82). Another attractive feature of these polymers is their mechanical strength which is important in augmenting load bearing bones. These polymers however, are not osteoin- ductive and in some cases their lack of porosity can obstruct bone penetration into the implant (A. Tencer et al. (1987) J. Orthop. Res., 5:275-282).

Demineralized bone matrix (DBM) is a bone derivative that is prepared by demineralizing cadaver bone with HCI. Various forms have been produced including powder, chips and blocks. DBM has been shown to exhibit osteoinduction (I.A. Guterman et al. (1988) Collagen Rel. Res., 8:419-431; A.H. Reddi and W.A. Anderson (1976) J. Cell Biol., 69:557-572; H.E. Firschein and M.R. Urist (1972) Clin. Orthop., 84:263-275) and clinically it is used primarily for facial skeletal augmentation and reconstruction (J.B. Mulliken et al. (1981) Ann. Surg., 194:366-372). In many cases, however, bone resorption often occurs with DBM implants. A recent study concluded that demineralized bone had an unacceptable high resorption rate and should only be used in cases where the implant is positioned in sites rich in primitive mesenchymal cells or bone-forming cells (D.M. Toriumi, et al. (1990) Arch. Otolaryngol Head Neck Surg, 116:676-680).

DBM implants do not possess the mechanical strength to be used in applications where the implant is exposed to stress. Stress resistance and weight bearing capacity can be critical for implants in the ap- pendicalar skeleton and mandible. Various materials have been combined with DBM to form composites that are capable of enhancing new bone formation while improving the mechanical properties of the implant. Natural composites include DBM plus autogenous bone marrow (E. Green, et al. (1986) Clin Orthop., 205:293-298; T.S. Lindholm and M.R. Urist (1980) Clin. Orthop., 150:288-300) and autogenous bone (P. Kohler and A. Kreicberg (1987) Clin. Orthop., 218:247-258). Several synthetic composites have also been made using porous polysulfone (J. J. Vam- dersteenhoven and M. Spector (1983) J. Biomed. Mater. Res., 17:1003-1014) and hydroxyapatite (S.G. Hopp et al. (1989) J. Orthop. Res., 7:579-584). Only limited success has been achieved with these systems.

In an effort enhance osteogenesis some focus has been directed toward understanding the underlying mechanisms that govern the regulation of healing. Growth factors are known to regulate the cellular functions of many processes, particularly the healing of tissue. Transforming growth factor-β (TGF-β), basic fibroblast growth factor (bFGF) and platelet derived growth factor (PDGF) are several polypeptide growth factors that are central to the tissue repair process. Among these proteins, the TGF-β superfamily appears to play a critical role in bone healing (G.F. Pierce et al. (1989) J. Cell Biol., 109:429-440).

TGF-β is a 25 kD homodimer̅i̅c̅ protein that stimulates the migration, proliferation, and matrix synthesis of mesenchymal cells. Bone is the largest reservoir of TGF-β in the body (S.M. Seydin et al. (1986), J. Biol. Chem., 261 :5693-5695), and its ability to promote bone formation suggests that is may have potential as a therapeutic agent in deseases of bone loss (M. Noda and J. Camilliere (1989) Endocrinology, 124:2991-2207). The in vivo application of TGF-β adjacent to periosteum has been shown to increase bone thickness and chondrogenesis (M.E. Joyce et al. (1990) J. Cell Biol., 110:2195; C. Marcelli et al. (1990) J. Bone Miner. Res., 5:1087-1096; E.J. Mackie and U. Trechsel (1990) Bo̅n̅e, 11:295-300). TGF-β has also been shown to induce bone closure of large bony defects in the skull (L.S. Beck et al. (1991) J. Bone and Miner. Res., 6:1257-1265).

Despite these promising findings, there exists a need for an effective method of delivering TGF-β to osseous defects to promote bone healing. The growth factor should be delivered in stable form without denaturation or loss of bioactivity. Also, there exists a need for a method of predictably controlling the amount and duration of TGB-β release from this system over a given time period.

Other protein delivery systems have been described which are made of non-degradable polymers (Folkman et al., U.S. Patent 4,164,560) or from degradable PLPG copolymers (Kent et al., Microencap- sulation of water soluble active polypeptides, U.S. Patent 4,675,189; Hutchinson, Continuous release pharmaceutical compositions, U.S. Patent, 4,767,628; Gombotz, et al., Very low temperature casting of controlled release microspheres, U.S. Patent 5,019,400). These types of systems however, lack either the biodegradability, mechanical properties, morphology or porosity necessary for successful skeletal augmentation. Therefore, an object of this present invention is to provide a method for making an implantable TGF-β delivery system that is capable of stimulating bone growth.

### SUMMARY OF THE INVENTION

The present invention is directed to a biodegradable composition for the release of TGF-β, an implantable delivery system for TGF-β release and a composition for stabilizing TGF-β activity.

The biodegradable composition of the present invention contains a mixture of demineralized bone matrix (DBM) and a degradable homopolymer or copolymer, such as poly (lactic-co-glycolic acid) (PLPG), in which TGF-β is dissolved or dispersed. This composition is capable of releasing TGF-β in a predictable and controlled manner with time. TGF-β can be released from this composition in either a bioactive or a latent form. Additional polymers or other excipients can also be added to this composition in order to vary the properties or release kinetics of the composition. The TGF-β released from this composition can be any member of the TGF-β family and particularly TGF-β₁, TGF-5β or bone morphogenetic protein.

The delivery system of this invention is composed of an implantable support that contains a biodegradable composition of the present invention. The composition can be casted or molded in a variety of shapes to form the support or the implantable support of this system can be a biodegradable or a solid non-degradable support coated with the biodegradable composition.

Acomposition forstabilizing TGF-β is further contemplated in this invention. This composition contains an organic acid buffer, such as citric acid or acetic acid, maintained at pH 2.5. This composition can also contain mannitol, human serum albumin, gelatin and/or a non-ionic detergent such as Tween-80^{TM}. This composition is maintained as either a solution or a lyophilized powder. When constituted as a solution about 30 mM citric acid, and about 30 mg/ml mannitol, and maintained at about pH 2.5, this composition is capable of maintaining TGF-β in concentrations of about 0.1 mg/ml to 1.0 mg/ml as an active molecule for extended periods of time (in excess of 6 months) at 4 °C.

When lower concentration of TGF-β (about 0.001 mg/ml) were utilized additional additives were supplied to the buffer solution, such as human serum albumin, gelatin and/or a nonionic detergent.

### BRIEF DESCRIPTION OF THE DRAWINGS

FIGURE 1 illustrates the cumulative percent of TGF-β₁ released from demineralized bone powder over time.
Figure 2 illustrates a schematic representation of a TGF-P/DBM/PLPG implant.
FIGURE 3 illustrates the cumulative percent of TGF-β₁ released from demineralized bone powder/PLPG implant over time.
FIGURE 4 illustrates the cumulative amount of TGF-β₁ released (µg TGF-β₁/g implant) from a demineralized bone powder/PLPG implant over time as a function of TGF-β₁ loading.
FIGURE 5 illustrates the cumulative amount of TGF-β₁ released (µg TGF-β₁/g implant) from a demineralized bone powder/PLPG implant over time as a function of DBM in the implant.
FIGURE 6 illustrates the cumulative percent of TGF-β₁ released over time from a demineralized bone powder/PLPG implant treated with various coatings.

### DESCRIPTION OF PREFERRED EMBODIMENTS

The present invention is directed to compositions and systems forthe delivery and stabilization of TGF-β.

A biodegradable composition of the present invention contains, at least, demineralized bone matrix and a degradable homopolymer or copolymer, such as poly (lactic-co-glycolic acid), in which TGF-β is dissolved or dispersed. This composition is capable of releasing TGF-β in a predictable and controlled manner with time.

As used herein "TGF-β" refers to a compound which is a 25kD homodimeric protein that stimulates the migration, proliferation and matrix synthesis of mesenchymal cells. Molecules which are members of the TGF-β superfamily are encompassed in this definition and include TGF-β₁, TGF-5β, and bone morphogenetic protein. TGF-5β is a hybrid molecule of TGF-β₁ and TGF-13₂ (Madisen et al. (1990) in Transforming Growth Factor-(3s Chemistry, Biology, and Therapeutics, Anal. NY Acad. Sci., 593:7-25).

The biodegradable composition of the present invention can also contain other polymers which will contribute to its structure and properties. Illustrative polymers that can be included within this composition are poly (lactic acid), poly (lactic-co-glycolic acid), poly (glycolic acid), poly (caprolactone), poly (hydroxybutyric acid), poly (anhydrides), poly (ortho esters), poly (carbonates), poly (amides), poly (acetals), poly (amino acids) and poly (cyanoacrylates). One or more of such polymers can be incorporated into the biodegradable composition of this invention. Additionally, additives can be included in the composition to vary mechanical properties or release kinetics. Illustrative additives includes plasticizers such as poly (oligo-L-lactide), triethyl citrate, collagen, or glycerol, as well as molecules of different molecular weights and types, such as lactide glycolide copolymers, which can be used to vary the strength and degradation rate of the composition.

The composition of the present invention can contain TGF-β in either a latent or bioactive form, as well as physiologically tolerable or pharmaceutically acceptable derivatives thereof.

As used herein, the phrases "physiologically tolerable" and "pharmaceutically acceptable" are used interchangeably and refer to molecular entities which are capable of administration to a mammal without producing untoward efforts not contemplated by the administration of the desired agent.

A carrier or matrix in the present invention is a material useful for administering an active compound and must be "pharmaceutically acceptable" in the sense of being compatible with the other ingredients of the composition and not deleterious to the recipient thereof.

The compositions of the present invention are prepared by any of the methods well known in the art, all of which involve bringing into association the active compound(s) and the carrier thereof. In these compositions, the TGF-β is typically dissolved or dispersed in a physiologically tolerable carrier.

It should be understood that in addition to the aforementioned components, the compositions described herein can contain, as appropriate, one or more additional ingredients such as diluents, buffers, binders, surface active agents, thickeners, lubricants, preservatives (including antioxidants) and the like.

Adelivery system of the present invention is composed of an implantable support which contains a biodegradable composition as described herein from which TGF-β is capable of release in a predictable and controlled manner with time.

The implantable support in the delivery system supplies a solid matrix which is capable of being inserted and/or molded into a portion of a mammalian body. This support can be a biodegradable support which, during and/or following the release of TGF-(3, slowly breaks down or is absorbed by the body, or a solid, nondegradable support, such as a prosthesis, which is coated with a biodegradable composition of the present invention. This system can also be composed of the biodegradable composition formed into an implantable support by solvent casting a compression molding.

Solvent casting is done by first dissolving the polymer in an appropriate solvent which is them mixed with the TGF-β and DBM in addition to other additives. The mixture is then cast in a mold to form the desired implant shape such as a cylinder or flat film. The solvent is the allowed to evaporate (or coated on another implant) from the implant. Solvent evaporation can be accelerated by subjecting the implant to vacuum.

Compression is done by first mixing the TGF-β/DBM with dried polymer particles. The dried mixture is then placed in a mold and compressed resulting in formation of the implant.

This delivery system enables the implantation of specific TGF-β releasing compositions in particular locations where the release of TGF-β can be controlled in a predictable manner.

The implantable system is capable of delivering TGF-β, in one embodiment, to a bone defect. It acts as a degradable scaffolding into which new bone growth can occur. The solid support is either coated with or composed of DBM that has been co-lyophilized with TGF-β and then incorporated into a PLPG matrix. This system releases TGF-β in either an active or latent form at different dosages and rates and possesses a porosity that is compatible with bone ingrowth.

In a preferred embodiment, the implantable system of this invention has mechanical properties and characteristics that enable it to be sutured, or otherwise inserted, into place in a body and to bear stress.

In one embodiment of the present invention, a biodegradable composition is formed by suspending DBM in a solution containing TGF-β. This suspension results in the incorporation of some TGF-β into the bone powder. The suspension is then lyophilized to produce a dry DBM powder containing adsorbed TGF-β. A PLPG solution is prepared by dissolving poly (lactic-co-glycolic acid) in a solvent, such as me- thylane chloride, acetone or chloroform.

The dried DBM powder containing adsorbed TGF-β is added to the PLPG solution to form a suspension. This suspension can be poured into a mold and dried, or it can be coated onto a implantable support. The biodegradable composition can also contain or be coated with polymers to modify the mechanical properties or release kinetics of the composition or delivery system.

In another embodiment, the implantable support can be prepared without solvents, such as by a compression molding technique.

The amount of TGF-β released and the release rate can be predictably and reliably controlled by varying eitherthe amount of TGF-β or a DB M incorporated into the system.

The combination of DBM and growth factors has been shown to stimulate bone growth (Toriumi et al. (1991) J. Long Term Effects on Med. Implants, 1:53-77). However, several shortcoming are present in the reported systems, such as very little control of the release kinetics of the growth factorfrom the bone powder.

Control of the growth factor delivery rate can be important in stimulating bone growth. The initial release of a small amount of TGF-β from an implant could act as a chemoattractant capable of recruiting osteoblasts to the site. Later recruitment of new cells would become less important than osteogenesis into or around the implanted support.

The biodegradable compositions and delivery systems of the present invention enable the precise control of the release kinetics of TGF-β and retain the bioactivity of the TGF-β. In a preferred embodiment, the described systems posses adequate strength to be sutured into place and to remain localized throughout the osteogenesis process.

A composition for stabilizing TGF-β has been formulated in the present invention. TGF-β1 has been isolated and purified in 5 mM HCI, pH 2.5. The molecule, however, is unstable in this solution over time. When maintained at 4°C, TGF-β1 preparations in 5 mM HCI display a continual increase in ascending peak size, when assayed by reverse phase high performance liquid chromatography (HPLC). Freezing of TGF-β₁ in this formulation causes significant aggregation, as detected by Western blotting.

The present inventor has found that lyophilization of both TGF-0₁ and TGF-5β under specific conditions is a viable method of stabilizing TGF-β from degradation and aggregation. When TGF-β₁ is lyophilized at concentrations greater than or equal to 0.1 mg/ml in about 10 to about 50 mM of an organic acid, such as citric acid or acetic acid, containing between about 20 to 40 mg/ml mannitol, at pH 2.5, the protein retains complete bioactivity when assayed by growth inhibition assays (GIA) or proliferation assays. The TGF-β also undergoes no reduction in concentration, as determined by ELISA.

Reverse phase HPLC analysis of both TGF-β₁ and TGF-5β stabilized in this composition indicated that both proteins were stable and did not undergo an increase in ascending peak size upon lyophilization or after storage for over six months. Western blot analysis showed that the proteins did not aggregate.

In a preferred embodiment TGF-β was further shown in this invention to be stable in a 30 mM citric acid buffer plus mannitol for over 60 days at 4°C. When frozen at-70°C in this buffer, TGF-β₁ was maintained without any noticeable degradation.

When TGF-β1 formulations at lower concentrations, such as 1 µg/ml were prepared in the cit- rate/mannitol buffer described above, significant losses in bioactivity and protein concentrations were fou nd, likely due toTGF-β₁ adsorption to vials in which is was stored. The addition of either about 6 mg/ml of human serum albumin (HSA) or gelatin to the formulation prevented this loss of TGF-β1. The use of siliconized glass vials and the addition of about 0.01% non-ionic detergent (Twenn-80^{TM}) to the composition prevented reductions in TGF-β₁ concentration and activity.

The present invention is further illustrated by the following Examples which are not intended to limit the scope of the invention.

### EXAMPLE 1

### Preparation of DBM Particles Containing TGF-β₁

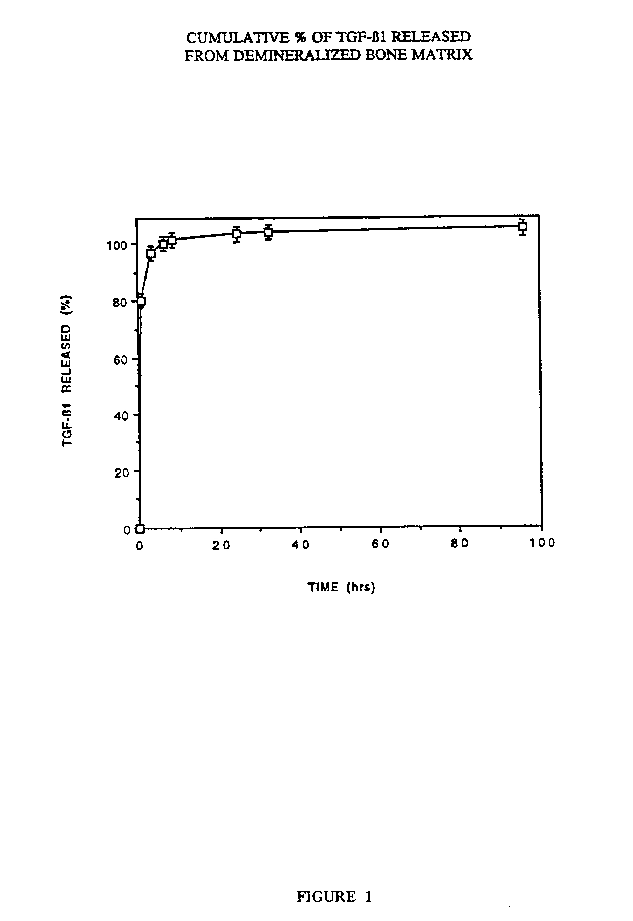

A 54 mg sample of powdered DBM (Northwest Tissue Center, Seattle, WA) was prepared by HCI extraction and lyophilization. The final preparation was a powder consisting of particles ranging in size from 74 µm to 640 µm. The DBM was added to 2 ml of a 30 mM sodium citrate buffer solution containing 30 mg/ml mannitol, pH 2.5. To this suspension was added 25 µg of TGF-p1 (Bristol-Myers Squibb Pharmaceutical Research Institute, Seatle) and 40 µl of ¹²⁵1- labeled TGF-β₁. The suspension was allowed to equilibrate for 12 hours at 4°C after which it was frozen in liquid nitrogen and lyophilized. The entire sample was then rehydrated in 4 ml phosphate buffered saline (PBS) containing 1 % human serum albumin (HSA) (Sigma Chemical Co.), pH 7.4 and incubated while shaking at 37°C. At various times the sample was centrifuged, the supernatant removed and replaced with new PBS. The amount of TGF-β present in the supernatant was determined by counting on a gamma counter. This was repeated at specified time points to quantitate the release of TGF-β1 from DBM. Figure 1 shows that all of the TGF-β₁ is release from the DBM within the first 10 hours.

### EXAMPLE 2

### Preparation Of a Buffer In Which TGF-13₁ Can Be Lyophilized And Remain Stable

TGF-β₁ was exchanged into a buffer made of 30 mM citric acid with 30 mg/ml mannitol at a pH of 2.5 using a Centricon 10 microconcentrator (Amicon). Different concentrations of TGF-β and TGF-5β were made ranging from 1.0 mg/ml to .001 mg/ml. Some of the solutions containing 0.001 mg/ml TGF-β contained additional additives including 6 mg/ml human serum albumin (Sigma), 6 mg/ml gelatin (Sigma) or 0.01 % of a nonionic detergent, Tween-80^{TM} (Sigma). Samples (1.0 ml) were placed in 2 ml glass vials and lyophilized. Various assays were used to assess the stability of the TGF-β including reverse phase HPLC, ELISA, Western blot gel analysis and a growth inhibition assay. The results of these studies indicate that a formulation containing 30 mM citric acid and 30 mg/ml mannitol, pH 2.5, stabilized the TGF-β at concentrations of 100 µg/ml or greater. At lower concentrations of 0.001 mg/ml additional additives were necessary to prevent adsorption of the TGF-β to the vial. Both TGF-β₁ and 5-β were stable in these lyophilized formulations for over 6 months of storage at 4°C. This example demonstrates that TGF-β can be stabilized by lyophilization and describes several formulation that can be used for this purpose.

### EXAMPLE 3

### Preparation Of DBM Particles Containing TGF-β₁ Which Is Incorporated Into A Poly(Lactic-Co-Glycolic Acid) Matrix

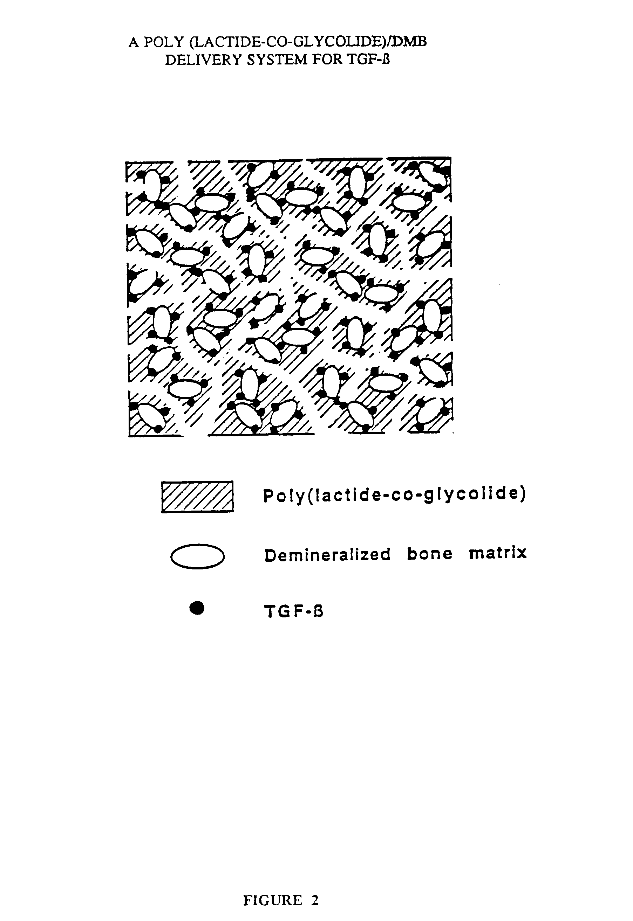

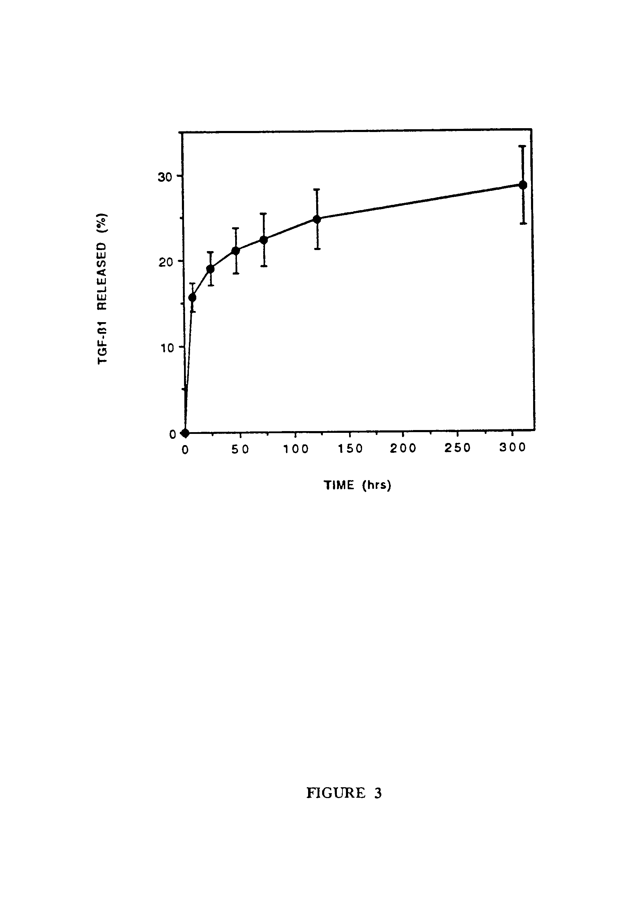

A 30% (w/v) solution of poly (lactic-co-glycolic) acid (PLPG) (50:50) (Birmingham Polymers, Inc. Birmingham, AL) in methylene chloride was made by adding 1.2 g of PLPG to 4 ml of solvent. 2.25 ml of this solution was added to a lyophilized DBM/TGF-β₁ sample prepared according to Example 1. The suspension was then poured into polypropylene molds and the solvent allowed to evaporate at room temperature for days. Additional removal of solvent was accomplished by placing the sample in a vacuum desiccator for 24 hours. The resulting macroporous matrix consisted of DBM/TGF-β₁ particles coated with PLPG. The devices contained approximately 26% DBM by weight. The morphology of the implants appeared to be sufficiently porous to allow bone ingrowth when analyzed by scanning election microscopy. The pores ranged in size from about 10 µM up to 300 µM. Aschematic representation of the implants is shown in Figure 2. Release of TGF-β₁ from the dried implants was determined. The results are shown in Figure 3. The implant is capable of controlling the release of TGF-β for more than 300 hours. This example demonstrates that the release of the TGF-β₁ from DBM can be slowed down significantly by incorporating the TGF-β₁ co-lyophilized with DBM into a PLPG matrix.

### EXAMPLE 4

### Preparation Of An Implant Containing Only TGF-β₁And PLPG

A 54 mg sample of TGF-β₁ was added to 2 ml of a 30 mM sodium citrate buffer solution containing 30 mg/ml mannitol, pH 2.5 and lyophilized. This was essentially the same preparation described in Example 1 without the DBM. The lyophilized TGF-β₁ was then suspended in a 30% PLPG solution in methylene chloride, cast in a mold and allowed to cure. The resulting implant separated upon drying and had little porosity when analyzed by scanning election microscopy. This example demonstrates that a PLPG implant containing TGF-β₁ without DBM does not form an intact implant and has undesirable morphological characteristics for a bone regeneration system.

### EXAMPLE 5

### Preparation Of DBM Particles Containing TGF-13₁ Which Are Incorporated Into A Poly (Lactic-Co-Glycolic Acid) Matrix While Varying The TGF-13₁ Loading

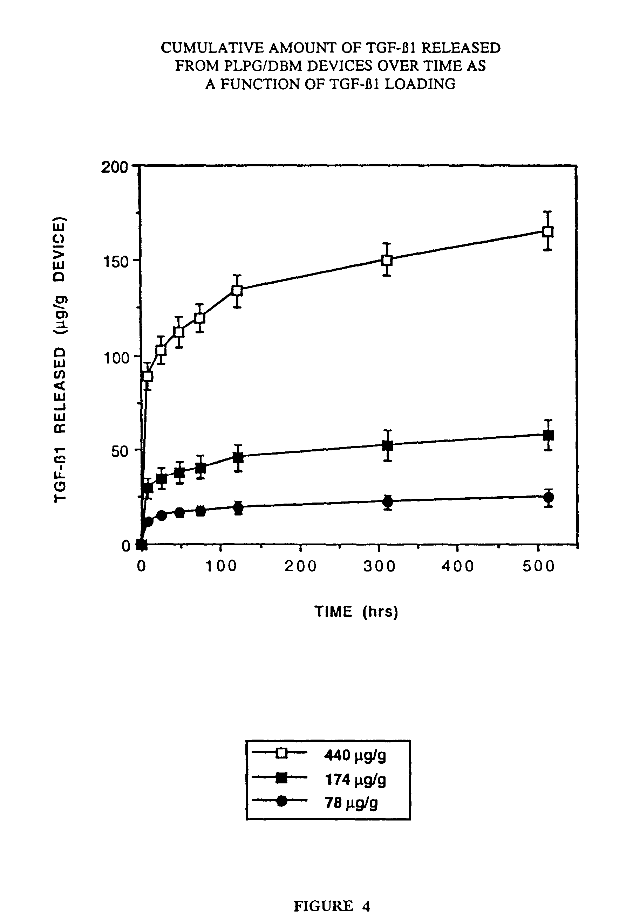

Three different co-lyophilized preparations of TGF-β₁ in DBM were prepared which contained varying ratios of TGF-β₁ to DBM (500 µg TGF-β₁/g DBM, 1000 µg/g and 2000 µg/g). These were cast into devices with PLPG as described in Example 4. The resulting devices contained 26% DBM by weight but were loaded with different amounts of TGF-β-₁. The amounts of TGF-β₁ per gram of device were 78 µg/g, 174 µg/g and 440 µg/g. TGF-β₁ was released from the different implants as shown in Figure 4. As the TGF-β₁ loading in increased, the amount of TGF-β₁ released over a given time period is also increased. Therefore, the amount of TGF-β₁ release from the implants can be controlled by varying the TGF-β₁ loading.

### EXAMPLE 6

### Preparation Of DBM Particles Containing TGF-13₁ Which Are Incorporated Into A Poly (Lactic-Co-Glycolic Acid) Matrix While Varying The DBM Loading

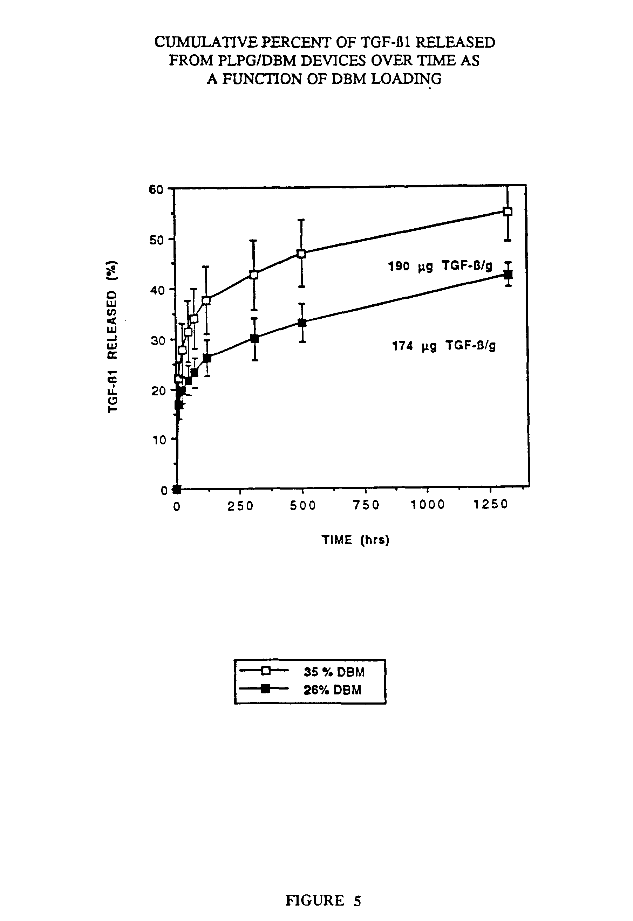

Two different TGF-β₁/DBM co-lyophilized samples were prepared which contained approximately equal amounts of TGF-β₁ but had different amounts of DBM. Implants were prepared as described in Example 4. The resulting devices contained 35% or 26% DBM by weight. Figure 5 shows the cumulative percent of TGF-β₁ released from the implants over time. Asthe DBM content of the implant was increased, the cumulative percent of TGF-β₁ released from the implant also increased. This example shows that the TGF-β release kinetics can be controlled by varying the DBM loading of the implant.

### EXAMPLE 7

### Preparation Of DBM Particles Containing TGF-13₁ Which Are Incorporated Into A Poly (Lactic-Co-Glycolic Acid) Matrix Which Is Then Coated With Different Polymers To Vary The Release Rate

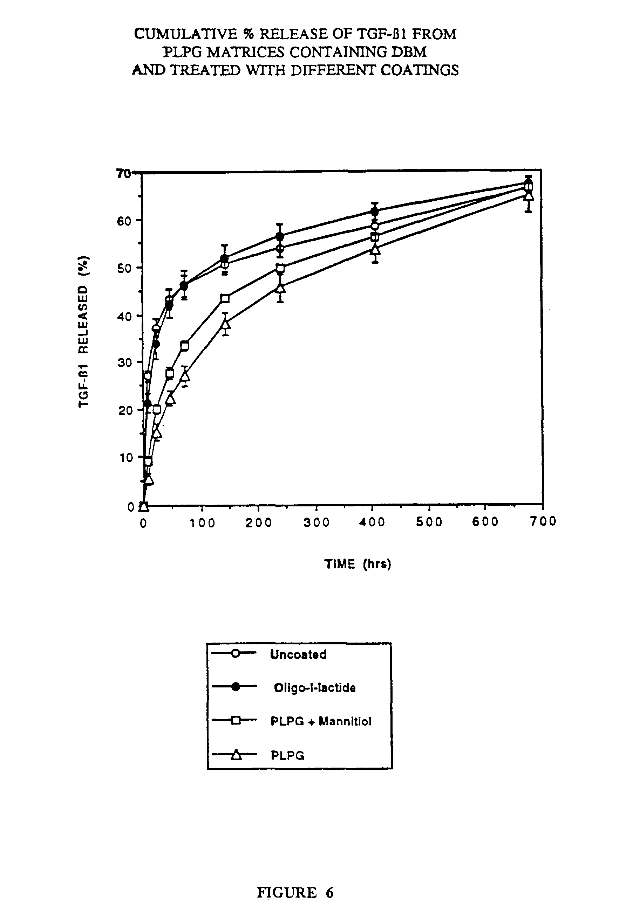

Implants were prepared as described in example 6 which contained approximately 26% DBM by weight and were loaded with 1000 µg TGF-β₁/g DBM. The implants were treated with various coating to further control the release kinetics. The coatings included a 10% PLPG solution, a 10% PLPG solution containing 20% mannitol (w/w) and a 10% oligo-L-lactide solution (Boehringer Ingelheim) all in methylene chloride. Coatings were done by first immersing the implants in liquid nitrogen and then dipping the implants into the coating solution. Samples were then allowed to air dry. The cumulative percent of TGF-β₁ released from these samples is shown in Figure 6. The most rapid release occurred in the uncoated and oligo-L-lactide coated devices. The oligo-L-Lactide decreased the amount released in the earlier time up to about 50 hrs after which time no significant decrease was observed. Coating the implant with a 10% PLPG solution on the other hand significantly decreased the initial amountof TGF-β₁ released . When mannitol was added to the PLPG coating, the TGF-β₁ release increased compared to the PLPG alone, probably due to the increased porosity of the PLPG/mannitol coating. This example indicates that the release kinetics of TGF-β₁ from the implant can be controlled, particularly at early time, by treating the implants with various coatings.

### EXAMPLE 8

### Determination Of the Binding Activity Of TGF-13₁ Released From A DBM/PLPG Implant Using ELISA

An implant was prepared as described in Example 4 without the addition of ¹²⁵1-labeled TGF-β₁. TGF-β was released into PBS containing 1% HSA for 48 hours and the concentration of material was determined by an enzyme linked immunosorbent assay (ELISA). The ELISA measures the ability of an anti TGF-β monoclonal antibody 1 D11 (prepared in house at Bristol-Myers Squibb, Seatle) to bind to the TGF-β₁ molecule. If the TGF-β does not retain its binding activity then it is good indication that the protein is inactive. The results of this study show that TGF-β₁ is released from the implant in a form which retains binding activity to a monoclonal antibody.

### EXAMPLE 9

### Determination Of the Bioactivity Of TGF-13₁ Released From A DBM/PLPG Implant Using Growth Inhibition Assay

Some of the buffer used in the ELISA described in Example 8 was retained for analysis in a growth inhibition assay (GIA). The GIA is used to measure the bioactivity of the TGF-β₁. Mink lung epithelial cells, Mv 1 Lu (Accession Number CCL-64, American Type Culture Collection), which are extremely sensitive to TGF-β were utilized for GIA. The assay was performed using the cellular conversion of a tetrazolium salt into a blue formazan product to assess cell viability. The results of this study indicate that all of the TGF-β₁ released from the implant as determined by ELISA, retained its bioactivity. This example demonstrates that TGF-β can be incorporated into and release from a DBM/PLPG implant in a bioactive form.

### EXAMPLE 10

### Preparation Of DBM Particles Containing TGF-13₁ Which Are Incorporated Into A Poly (Lactic-Co-Glycolic Acid) Matric along With Other Additives

An implant was prepared according to Example 4 with the addition of 2% by weight oligo-L-lactide (Boehringer Ingelheim). After the sample had been dried it was found to be much easier to remove from the mold and was more flexible than similar implants without the oligo-L-lactide. This example demonstrate that the implants can be made with the addition of other additives such as oligo-L-lactide, collagen or glycerol to modify the mechanical properties or releases that this brief list of additives is not limiting and many other synthetic or natural materials could be used.

### EXAMPLE 11

### Preparation Of DBM/PLPG Implant Containing TGF-β₁ By Compression Molding

A co-lyophilized preparation of TGF-β₁ in DBM (1000 µg/g) was prepared as described in Example 5. This material was then manually ground and mixed with a mortar and pestle with a dried PLPG, resulting in a final mixture containing 26% DBM by weight. The mixture was pressed into a circular disc using a Carver Laboratory Press Test Cylinder at 25,000 psi and room temperature for 10 min. The resulting device was intact and easily handled. The release of TGF-β₁ from this device was determined and was much faster than from a similar solvent cast device. This example shows that a solvent free compression molding technique can be used to make an TGF-β delivery device.

### EXAMPLE 12

### Measuring The Total Protein Release From A DBM/PLPG Delivery Device Containing TGF-13₁

An implant was prepared according to Example 4 and the TGF-β was released into PBS with no HSA. The total amount of protein present in the release buffer was determined using a bicinchoninic acid (BCA) assay (Pierce,. Rockford, IL). The assay is sensitive to protein concentrations of20 µg/ml and would therefore unable to detect the TGF-β₁ in the buffer. It significant degradation of the protein components in DBM were occurring they would be detected. The BCA analysis revealed that no detectable DBM degradation had occurred. This example demonstrates that the release ofTGF-β₁ from the implant is notcon- trolled by degradation of the DBM.

The foregoing description and the Examples are intended as illustrative and are not to be taken as limiting. Still other variations within the spirit and scope of this invention are possible and will readily present themselves to those skilled in the art.

## Claims

1. A biodegradable composition for the delivery of TGF-β comprising a mixture of demineralized bone matrix and a degradable homopolymer or copolymer, in which said TGF-β is dissolved or dispersed, and which is capable of a predictable and controlled release of TGF-β with time.

2. The composition according to Claim 1 in which TGF-β is released in a bioactive form.

3. The composition according to Claim 1 wherein the degradable homopolymers or copolymers is selected from the group consisting of poly (lactic acid), poly (lactic-co-glycolic acid), poly (glycolic acid), poly (caprolactone), poly (hydroxybutyric acid), poly (anhydrides), poly (ortho esters), poly (carbonates), poly (amides), poly (acetals), poly (amino acids) and poly (cyanocrylates).

4. The composition according to Claim 1, wherein said TGF-β is TGF-β₁.

5. The composition according to Claim 1, wherein said TGF-β is TGf-513.

6. The composition according to Claim 1, wherein said TGF-β is bone morphogenetic protein.

7. The composition according to Claim 1, where said mixture further comprises an additive which acts as a plasticizer or modifies the release kinetics of TGF-β from said composition.

8. The composition according to Claim 7, wherein said additive is an oligo-L-lactide.

9. The composition according to Claim 7, wherein said additive is collagen.

10. The composition according to Claim 7, wherein said additive is glycerol.

11. The delivery system for TGF-β comprising an implantable support coated with a biodegradable composition comprising a mixture of demineralized bone matrix and poly (lactic-co-glycolic acid) in which said TGF-ß is dissolved or dispersed, and which is capable of a predictable and controlled release of TGF-β with time.

12. The delivery system according to Claim 11, wherein said support is a biodegradable support.

13. The delivery system according to Claim 11, wherein said support is a non-degradable solid support.

14. The delivery system according to Claim 11, wherein said system comprises said biodegradable composition formed into an implantable support by solvent casting or compression molding.

15. A composition for stabilizing TGF-β comprising about 10 to about 50 mM of an organic acid buffer maintained at about pH 2.5

16. The composition according to Claim 15, wherein the buffer is a citric acid buffer.

17. The composition according to Claim 15, wherein the buffer is an acetic acid buffer.

18. The composition according to Claim 15, wherein the composition contains at least one additional component selected from the group consisting of mannitol, human serum albumin, gelatin and a nonionic detergent.

19. The composition according to Claim 15, wherein said composition is a lyophilized formulation capable of reconstruction.
